Europäisches Patentamt

European Patent Office

Office européen des brevets

(11) Veröffentlichungnummer: **0 340 593**

**A2**

(12) **EUROPÄISCHE PATENTANMELDUNG**

(21) Anmeldenummer: 89107374.4

(22) Anmeldetag: 24.04.89

(51) Int. Cl.⁴: **C07B 41/04** , //C07C43/11, C07C41/03

(30) Priorität: 02.05.88 DE 3814849

(43) Veröffentlichungstag der Anmeldung:
08.11.89 Patentblatt 89/45

(84) Benannte Vertragsstaaten:
AT BE CH DE ES FR GB IT LI NL SE

(71) Anmelder: **Henkel Kommanditgesellschaft auf Aktien**
**Postfach 1100 Henkelstrasse 67**
**D-4000 Düsseldorf 1(DE)**

(72) Erfinder: **Ploog, Uwe, Dr.**
**Haydnweg 6**
**D-5657 Haan(DE)**

(54) **Verwendung von Estern der Titan- und/oder Zirkonsäure als Katalysatoren für die Ethoxylierung bzw. Propoxylierung.**

(57) Ester der Titan- und/oder Zirkonsäure mit Monoalkanolen mit 1 - 4 Kohlenstoffatomen in Kombination mit Schwefelsäure und/oder Alkansulfonsäure mit 1 - 6 Kohlenstoffatomen und/oder Hydroxyarylsulfonsäuren als Katalysatoren für die Ethoxylierung bzw. Propoxylierung von Verbindungen mit aktiven H-Atomen ergeben eine enge Homologenverteilung der Alkoxylierungsprodukte.

EP 0 340 593 A2

## Verwendung von Estern der Titan- und/oder Zirkonsäure als Katalysatoren für die Ethoxylierung bzw. Propoxylierung.

Die Erfindung betrifft Katalysatoren für die Ethoxylierung bzw. Propoxylierung von Verbindungen mit aktiven H-Atomen.

Unter Verbindungen mit aktiven H-Atomen im Sinne der Erfindung sind z.B. Fettalkohole, Guerbetalkohole, Fettsäuren und Amine zu verstehen, die bei der Ethoxylierung bzw. Propoxylierung nicht-ionische Detergentien bilden. Ein typisches Beispiel hierfür ist die Umsetzung von Fettalkoholen mit üblicherweise 10 bis 18 Kohlenstoffatomen mit Ethylenoxid und/oder Propylenoxid in Gegenwart von Katalysatoren, wobei die Fettalkohole mit mehreren Molekülen Ethylenoxid und/oder Propylenoxid reagieren.

Als Katalysatoren für die vorgenannte Polyalkoxylierung sind hierfür u.a. die folgenden eingesetzt worden:

Calcium- und Strontiumhydroxide, -alkoxide und phenoxide (EP-A 00 92 256),

Calciumalkoxide (EP-A 00 91 146),

Bariumhydroxid (EP-B 0 115 083),

basische Magnesiumverbindungen, z.B. Alkoxide (EP-A 00 82 569),

Magnesium- und Calciumfettsäuresalze (EP-A 0 85 167).

Antimonpentachlorid (DE-A 26 32 953),

Aluminiumisopropylat/Schwefelsäure (EP-A 22 81 21).

Gebräuchliche Polyalkoxylierungskatalysatoren sind weiterhin Kaliumhydroxid und Natriummethylat.

Die vorgenannten Katalysatoren weisen u.a. den Nachteil auf, daß sie schlecht in das Reaktionssystem einarbeitbar und/oder schwierig herstellbar sind. Weiterhin ist bei der Verwendung von Alkalihydroxiden sowie von Alkalialkoholaten die erreichbare Bandbreite des Polyalkoxylierungsgrades, d.h. die Homologenverteilung, auf die nachfolgend näher eingegangen wird, groß bzw. breit. Saure Katalysatoren, z.B. Antimonpentachlorid, bewirken zwar eine engere Homologenverteilung und niedrigeren Fettalkoholgehalt der Reaktionsprodukte, zeigen jedoch hohe Korrosivität und sind teilweise toxisch. Bei der Verwendung von Gemischen aus Aluminiumalkoholaten und Schwefelsäure läßt sich zwar bei linearen Fettalkoholen eine enge Homologenverteilung der Alkoxylierungsprodukte bei gutem Umsatz erreichen, nicht jedoch bei verzweigten Alkoholen, z.B. den sogenannten Guerbet-Alkoholen.

Für Fettalkoholpolyalkoxylate ist die sogenannte enge Bandbreite des Polyalkoxylierungsgrades von besonderer Bedeutung, vgl. JAOCS, Vol. 63, 691 - 695 (1986), und HAPPI, 52 - 54 (1986). Die sogenannten "narrow-range"-Alkoxylate weisen demnach insbesondere die folgenden Vorteile auf:

- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch
- Reduzierung des Plumings beim Sprühtrocknen von Waschmittelslurries, die Fettalkoholpolyalkoxylat-Tenside enthalten.

Die Bandbreite bzw. Homologenverteilung der Fettalkoholpolyalkoxylate hängt wesentlich von der Art des verwendeten Katalysators ab. Als Maß für die Homologenverteilung gilt der sogenannte Q-Wert gemäß der Beziehung

$$Q = n^* \cdot p^2$$

in der $n^*$ die durchschnittliche Adduktzahl (mittlerer Ethoxylierungsgrad) und p den Prozentsatz des Adduktes mit bestimmtem EO-Grad, das überwiegend gebildet wird, bedeuten. Ein großer Q-Wert bedeutet somit eine enge Bandbreite der Homologenverteilung.

Oxethylate mit einem niedrigen Gehalt an freien Fettalkoholen weisen bei der Herstellung von Ethercarbonsäuren durch Umsetzung der Oxethylate mit Natrium-chloracetaten in Gegenwart von Alkalihydroxiden einen erhöhten Umsetzunggrad auf. Weiterhin sind Oxethylate mit eingeschränkter Homologenverteilung höchst wirksame Verdickungsmittel für Tensidlösungen.

Es wurde nun gefunden, daß man unter erfindungsgemäßer Verwendung von Estern der Titan- und/oder Zirkonsäure mit Monoalkanolen mit 1 bis 4 Kohlenstoffatomen zusammen mit Schwefelsäure und/oder Alkansulfonsäuren mit 1 bis 6 Kohlenstoffatomen und/oder Hydroxyarylsulfonsäuren als Katalysatoren für die Ethoxylierung bzw. Propoxylierung von Verbindungen mit aktiven H-Atomen zu einer engen Homologenverteilung der Polyalkoxylierungsprodukte kommt, wobei der Gehalt an freien Fettalkoholen in den Reaktionsprodukten gegenüber gebräuchlichen Katalysatoren deutlich herabgesetzt ist; es lassen sich im übrigen auch verzweigte Fettalkohole wie die vorgenannten Guerbet-Alkohole problemlos polyalkoxylieren.

Die erfindungsgemäß einzusetzenden Ester der Titan- und/oder Zirkonsäure mit Monoalkanolen mit 1 bis 4 Kohlenstoffatomen sind bekannte und im Handel erhältliche Verbindungen; typische Beispiele sind Titantetramethylat, -ethylat, -n-propylat, -i-propylat und -i-butylat sowie die entsprechenden Zirkonester.

Erfindungsgemäß werden die Ester der Titan- und/oder Zirkonsäure mit Monoalkanolen mit 1 bis 4 Kohlenstoffatomen in Mischung mit Schwefelsäure und/oder Alkansulfonsäuren mit 1 bis 6 Kohlenstoffatomen und/oder Aryloxysulfonsäuren eingesetzt. Dabei wirken sowohl die Titan- und/oder Zirkonester als auch Schwefelsäure bzw. die genannten Sulfonsäuren für sich allein bei praktikablen Konzentrationen nicht als Katalysatoren; der gewünschte katalytische Effekt tritt erst bei geeigneter Kombination dieser Komponenten auf. Als Alkansulfonsäuren mit 1 bis 6 Kohlenstoffatomen eignen sich z.B. Methan-, Ethan-, Propan-, Butan-, Pentan- und Hexansulfonsäuren. Ein typisches Beispiel für eine ebenfalls geeignete Hydroxyarylsulfonsäure ist Phenolsulfonsäure.

Gemäß einer bevorzugten Ausführungsform des Verfahrens der Erfindung setzt man die Katalysatoren in einer Menge von 0,2 bis 2 Gew.-%, bezogen auf das Endprodukt der Ethoxylierung bzw. Propoxylierung, ein.

Gemäß einer weiteren vorteilhaften Ausführungsform der Erfindung setzt man die Ester der Titan- und/oder der Zirkonsäure einerseits sowie Schwefelsäure und/oder Alkansulfonsäuren mit 1 bis 6 Kohlenstoffatomen und/oder Hydroxyarylsulfonsäuren anderseits in molaren Verhältnissen zueinander von 1:1 bis 1:4 ein.

Die Erfindung wird im folgenden anhand von bevorzugten Ausführungsbeispielen näher erläutert.

1. Herstellung der Katalysatoren.

Die Katalysatorkombinationen werden zweckmäßigerweise durch Vermischung der in Tabelle 1 aufgeführten Mengen mit dem zu polyalkoxylierenden Material, insbesondere den Fettalkoholen gemäß Tabelle 1, hergestellt. Man kann jedoch den Katalysator z.B. in Form einer konzentrierten alkoholischen Ausgangslösung herstellen, von der aliquote Teile zum Einsatz gelangen.

2. Bedingungen der Oxalkylierung.

Die zu oxalkylierenden Fettalkohole gemäß Tabelle 1 wurden mit den gegebenen Katalysatorkombinationen versetzt und in einen geeigneten Autoklaven überführt. Nach ca. 30 min Evakuierung bei 100° C und Spülung mit Stickstoff wurde die Temperatur auf ca. 180° C gesteigert und die gewünschte Menge Ethylenoxid bei maximal 5 bar aufgedrückt. Nach einer Nachreaktionszeit von 30 min wurde nicht umgesetztes Alkylenoxid bei 100° C/14 mbar abgezogen.

Von den gebildeten Alkoxylaten wurden die OH-Zahl und der Polyglykolgehalt (PG) bestimmt; weiterhin wurde ein Gaschromatogramm zur Ermittlung der Homologenverteilung bzw. des Q-Wertes anhand der gemessenen Flächenprozente erstellt.

Beispiele 1 bis 6.

In diesen Beispielen wurde nach der obigen Arbeitsvorschrift zur Ethoxylierung von Fettalkoholen mit 2 Mol Ethylenoxid verfahren. In Tabelle 1 sind die eingesetzten Fettalkohole und Katalysatoren, die eingesetzte Katalysatormenge (in mMol/100 g Ethoxylierungsprodukt), die Reaktionszeit, die Reaktionstemperatur, der ermittelte Q-Wert, die OH-Zahlen der Ethoxylierungsprodukten (ist/soll), der Gehalt an freien Fettalkoholen (FFA, %) und der Polyglykolgehalt (PG, %) zusammengefaßt.

Vergleichsversuche

Die Ergebnisse mit aus dem Stand der Technik bekannten Katalysatoren sind in Tabelle 2 zusammengefaßt.

Die in den Tabellen erwähnten Fettalkohole waren handelsübliche, technische Fettalkoholgemische der folgenden Zusammensetzung:

C 12/14: Fettalkohol der Kettenlängenverteilung 0 - 2 % C 10, 70 - 75 % C 12, 20 - 30 % C 14, 0 - 2 % C 16; Jodzahl kleiner als 0,3, durchschnittliches Molekulargewicht 194, Hydroxylzahl 285 - 293.

3

C 12: Fettalkohol der Kettenlängenverteilung 0 - 2 % C 10, mindestens 97 % C 12, 0 - 3 % C 14, Jodzahl kleiner als 0,3, durchschnittliches Molekulargewicht 188, Hydroxylzahl 295 -301.

Guerbet-C 16: 2-Hexyldecanol mit einer Hydroxylzahl von 190 -230, einer Jodzahl von weniger als 10 und einer Viskosität bei 20° C von ca. 40 mPa.s.

## Tabelle 1

| Ethoxylierung von Alkoholen mit 2 Mol Ethylenoxid | | | | | | | | |
|---|---|---|---|---|---|---|---|---|
| Beispiel Nr. | Alkohol | Katalysator | Kat.menge/mMol/100 g Produkt | Rkt.zeit (h)/-temp. (°C) | Q | OH-Zahl ist/soll | FFA (%) | PG(%) |
| 1 | C 12/14 | $Ti(OiC_3H_7)_4$/ $H_2SO_4$ | 2,7 5,7 | 5/180 | 2258 | 220/204 | 16 | 1,2 |
| 2 | C 12 | $Ti(OiC_3H_7)_4$/ $HO-C_6H_4-SO_3H$ | 5 10 | 5,5/150 | 2436 | 232/204 | 16,7 | 1,6 |
| 3 | C 12 | $Ti(OiC_3H_7)_4$/ $H_2SO_4$ | 4,4 2,2 | 8/140 | 1700 | 218/204 | 12,5 | 2,7 |
| 4 | C 12 | $Zr(OiC_3H_7)_4$/ $CH_3SO_3H$ | 5 10 | 4/140 | 1682 | 216/204 | 9,8 | 0,3 |
| 5 | Guerbet-C 16 | $Ti(OiC_3H_7)_4$/ $CH_3SO_3H$ | 4,5 9,1 | 7/180 | 899 | 147/155 | 17,9 | 10 |
| 6 | Guerbet-C 16 | $Ti(OiC_3H_7)_4$/ $CH_3SO_3H$ | 9,0 | 3/180 | 924 | 163/155 | 20,4 | n.b. |

## Tabelle 2

| Ethoxylierung von Alkoholen mit 2 Mol Ethylenoxid (Vergleichsversuche) | | | | | | | |
|---|---|---|---|---|---|---|---|
| Fettalkohol | Katalysator | Kat.menge (mMol/100 g Produkt) | Rkt/zeit (h)/-temp. (°C) | Q | OH-Zahl ist/soll | FFA (%) | PG (%) |
| C 12/14 | $NaOCH_3$ | 2 | 1/180 | 655 | 204/204 | 31,9 | 0,3 |
| Guerbet - C 16 | $NaOCH_3$ | 2 | 3/180 | 225 | 164/155 | 40,2 | 4,1 |
| C 12 | $SbCl_5$ | 1,7 | 5/180 | 1972 | 208/204 | 8,3 | 1,2 |
| C 12 | $Al(OiC_3H_7)_3$/ $H_2SO_4$ | 4,4 2,2 | 2,5/140 | 3330 | 216/204 | 16 | 4,6 |
| Guerbet - C 16 | $Al(OiC_3H_7)_3$/ $H_2SO_4$ | 4,9 2,5 | 3/140 | 880 | 178/155 | 49 | 11,3 |

## Ansprüche

1. Verwendung von Estern der Titan- und/oder Zirkonsäure mit Monoalkanolen mit 1 - 4 Kohlenstoffatomen zusammen mit Schwefelsäure und/oder Alkansulfonsäuren mit 1 - 6 Kohlenstoffatomen und/oder Hydroxyalkylsulfonsäuren als Katalysatoren für die Ethoxylierung bzw. Propoxylierung von Verbindungen mit aktiven H-Atomen.

2. Verwendung nach Anspruch 1, dadurch gekennzeichnet, daß man die Katalysatoren in einer Menge von 0,1 - 2 Gew.-%, bezogen auf das Endprodukt der Ethoxylierung bzw. Propoxylierung, einsetzt.

3. Verwendung nach Anspruch 1 oder 2, dadurch gekennzeichnet, daß man die Ester der Titan- und/oder der Zirkonsäure einerseits sowie Schwefelsäure und/oder Alkansulfonsäuren mit 1 - 6 Kohlenstoffatomen und/oder Hydroxyarylsulfonsäuren andererseits in molaren Verhältnissen zueinander von 1:1 bis 1:4 einsetzt.